# EUROPEAN PATENT APPLICATION

(11) **EP 1 792 595 A1**
(43) Date of publication of application: **06.06.2007**
(21) Application number: 05026305.2
(22) Date of filing: 02.12.2005
(51) Int. Cl.: A61G 1/06, A61G 1/02, A61G 12/00, B62D 1/28

(54) **Self-propelled patient transport system**

(71) Applicant: PAUL SCHERRER INSTITUT, 5232 Villigen PSI (CH)
(72) Inventor: Lempen, Daniel, 5016 Obererlinsbach (CH); Rhiner-Rosansky, Petra, 4800 Zofingen (CH); Flück, Peter, 3422 Kirchberg (CH)
(74) Representative: Fischer, Michael

(57) **Abstract**

The present invention provides a self-propelled patient transport system (2), comprising:
a) a patient table (6,6a,6b);
b) one self-propelled support vehicle (14) having means for loading or unloading a patient table;
c) a patient diagnosis and/or therapy station (10,12); and
d) a control means (28,30) for controlling the operation of the at least one self-propelled support vehicle (14); wherein
e) a patient disposed on the patient table is presentable at the patient diagnosis and/or therapy station (10,12) by controllably moving the support vehicle (14); and
f) said means for unloading a patient table (6,6a,6b) are provided to unload the patient table from the support vehicle (14) into a determined position within the patient diagnosis and/or therapy station and/or said means for loading the patient table (6,6a,6b) are provided to load the patient table (6,6a,6b) from that determined position into a determined position on the support vehicle (14).

By having the concept of the support vehicle which receives end discharges the patient table the patients can be prepared in a slot-like chain according the treatment schedule chosen for the diagnosis and/or therapy section which allow a high throughput of patients. The high throughput is not only a benefit for the provider, such as a hospital or a medical institute, but also for the patients since more patients can be treated within the operating time of the diagnosis and/or therapy section.

## Description

The present invention relates to a patient transport system.

In a medical environment patients have to be transported from their sick rooms to various patient diagnosis and/or therapy sections. Patient diagnosis sections are for instance all sections related to radiologic investigations, such as X-rays, computer tomography, angiography, nuclear medicine and the like. Accordingly, patient therapy sections are for instance the operating section and any type of particle and/or photon beam therapy sections, such as positron emission therapy, proton emission therapy, photon emission therapy and the like.

So far, movable sick beds exist which allow to transport the patient in his sick bed self-propelled and electrically driven to the desired destination section. Unfortunately, in most applications the patient can not be kept positioned in his sick bed which usually requires a lot of efforts and time to install the patient at the respective diagnosis and/or therapy section.

Considering the cost and the amortization requirements for the medical diagnosis and/or therapy equipment, it is of course highly desirable to arrive at a high throughput of patients in the respective diagnosis and/or therapy sections which is not supported yet by the existing patient transporting means, even they comprises electrical drives.

Therefore, it is the aim of the present invention to provide a patient transport system which allows to offer a high comfort and convenience (as far as this is possible in a medical environment) for the patient and simultaneously to support the demand for high throughput rates at the respective generally cost-intensive diagnosis and/or therapy sections.

These objectives are achieved according to the present invention by a remote-controlled patient transport system, comprising:
a) at least one patient table;
b) at least one self-propelled support vehicle having means for loading a patient table and/or means for unloading a patient table;
c) a patient diagnosis and/or therapy station;
d) a control means for controlling the operation of the at least one self-propelled support vehicle; wherein
e) a patient disposed on the patient table is presentable at the patient diagnosis and/or therapy station by controllably moving the support vehicle; and
f) said means for unloading a patient table are provided to unload the patient table from the support vehicle towards a determined position within the patient diagnosis and/or therapy station and/or said means for loading the patient table are provided to load the patient table from the determined position within patient diagnosis and/or therapy station towards a determined position on the support vehicle.

This patient transport systems allows to position a patient on a patient table and to transport the patient table supported on the support vehicle to the intended diagnosis and/or therapy station where the patient table is discharged from the support vehicle into a predetermined position within the diagnosis and/or therapy section. Additionally, after the diagnosis and/or therapy treatment, the patient table is again received on the support vehicle and subsequently the support vehicle is directed back to a preparation section or to an after treatment section where the patient can finally leave the patient table and return to his sick room either by feet or in his sick bed. By having the concept of the support vehicle which receives and discharges the patient tables the patients can be prepared in a slot-like chain according the treatment schedule chosen for the diagnosis and/or therapy section which allow a high throughput of patients. The high throughput is not only a benefit for the provider, such as a hospital or a medical institute, but also for the patients since more patients can be treated within the operating time of the diagnosis and/or therapy section.

In order to offer a high convenience for the patient, a-patient loading station can be provided which comprises means for disposing the patient table in different heights.

With respect to, for example, tumor treatments the patient has to be oriented and positioned extreme precisely relative to the photon and/or particle beam which interferes according to the predetermined treatment routine with the malicious tissue. Therefore, the discharge means can suitably comprise positioning aids in order to shift the patient table into the determined position on the support vehicle (when receiving the patient table) as well as in the preparation station and/or the patient diagnosis and/or therapy station. Suitable means can be for instance dovetailed rails or nuts or the like which support to link the patient table into the desired position.

In order to monitor the status of the patient, for instance the anesthesist who would like to monitor the status of a child being tranquilized for a radiation cycle, monitoring and/or patient supply equipment can be supported by the patient table and/or the support vehicle. Additionally, the patient table comprises electrical supply and connecting means in order to be supplied with electric energy in the patient preparation station and/or while the patient table is transported on the support vehicle and/or in the patient diagnosis and/or therapy station. Therefore, the patient table and the equipment disposed thereon can be operated independently from an individual direct power access.

For achieving a higher level of automation with the system, the support vehicle comprises guidance means in order to commute self-guided to a determined position. In particular, these guidance means may comprise path finding sensors which interact with respective landmarks positioned along the commuting ways. Alternately, the guidance means may comprise acceleration and direction sensors and/or wireless navigation sensors in order to self-guiding the support vehicle along the desired commuting path.

Again in order to support the observation of the demand for positioning the patient extreme precisely at least during the treatment cycle or session, the patient table may comprise control means for monitoring the position of the patient disposed on the patient table. Accordingly, the control means may be position sensors using ultrasonic position detection and/or using optical position detection and/or using RFID proximity detection, preferably in concert with respective landmarks positioned on the patient.

Since during the treatment session, such as a or several proton beam scanning cycles, the medical personal is absent in the direct treatment area in order to avoid collateral harmful effects like radiation pollution on the personal it is very important to make sure that the patient's position is not only monitored but measures are taken accordingly when the patient has moved (or for instance when organ movements are monitored) to a harmful extent. Therefore, the signals from the position sensors are checked against predetermined threshold value whereby diagnosis and/or therapy application is suspended in case one or more threshold values are exceeded or other predetermined condition are violated.

Examples of the present invention is described in detail hereinafter which reference to the following drawings.

Figure 1 illustrates a schematic overview on the remote controlled patient transport system.

Figure 2 illustrates a side view on a patient table disposed in the station where a patient enters the patent table.

Figure 3 depicts now in more detail a perspective front view on the support vehicle loaded with a patient table.

Figure 4 illustrates the support vehicle from Figure 3 in a perspective side view.

Figure 5 shows in a perspective view the support vehicle from Figures 3 and 4 now unloading the patient table to a pivot arm of a gantry system.

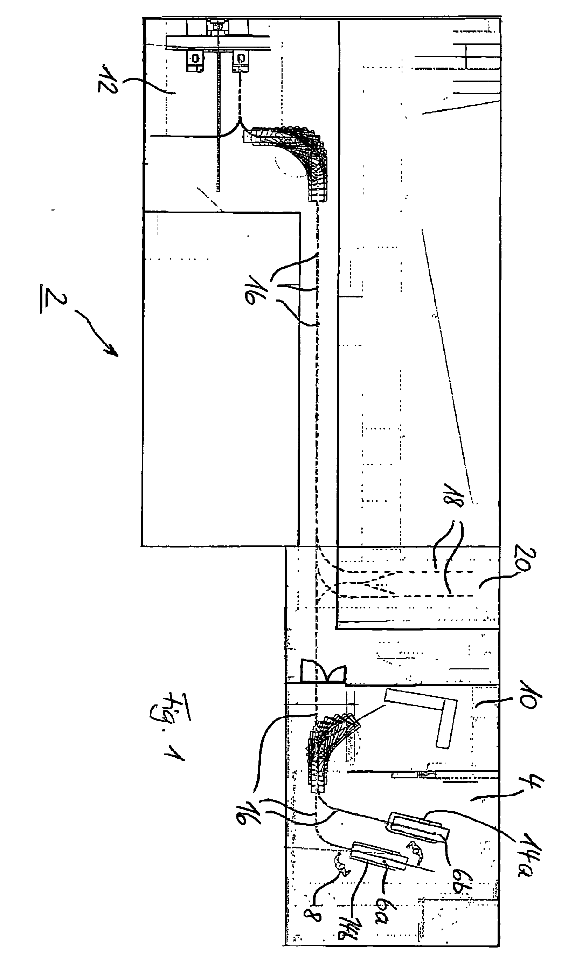

Figure 1 illustrates a schematic overview on a remote controlled patient transport system 2. The system 2 comprises a preparation section 4 where a patient can enter a patient table 6a, 6b (with support of medical personnel 8). Medical equipment in this area comprises a diagnosis section, hereinafter referred to as CT section 10, and a therapy section 12, hereinafter referred to as gantry section 12 using intensity modulated proton beam treatment. When the patient is disposed on one of the patient tables 6a or 6b, a support vehicle 14a, 14b which supports the patients tables 6a and 6b can self-propelled and remotely-controlled drive the patient along path landmarks 16 to the intended treatment section, either the CT section 10 or the Gantry section 12. Optionally, the patients can be parked in a waiting section 20 where two parking positions 18 are offered. Of course, the waiting section 20 can be also used as an after-treatment room into which the patient is transported after treatment, for instance to monitor the patient to recover from anesthesia.

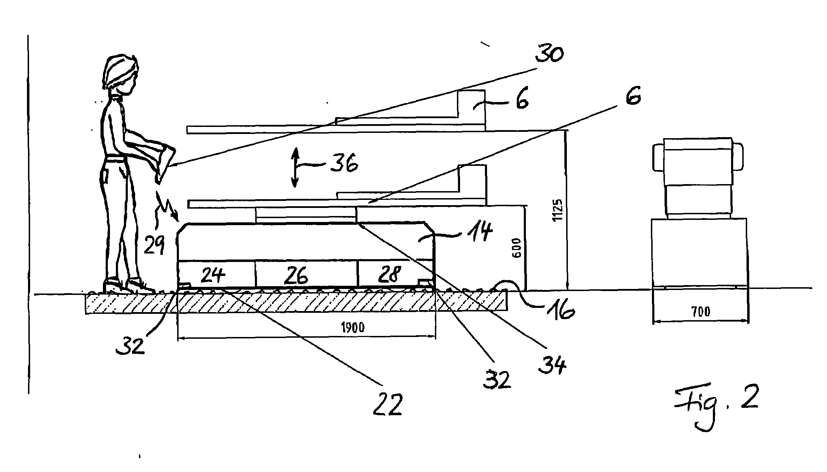

Figure 2 now illustrates a schematical side view on a support vehicle 14 and a patient table 6. The support vehicle 14 comprises a set of electrically driven rolls 22, a power support unit 24, medical equipment, such as an ECG device 26, and a control unit 28 which interacts by wireless communication 29 with a remote control unit 30. The medical personnel 8 controls with the remote control unit 30 the operation of the support vehicle 14 which is able to be self-guided along the landmarks 16 indicating the commuting paths. Position sensors 32 support the operation of the support vehicle 14 by interacting with the landmarks 16.

At the CT section 10 and/or the Gantry section 12 and/or at the waiting section 20 the patient table 6 can be discharged to a determined position in the respective treatment section. Therefore, a mechanical coupling and load mechanism 34 is provided to either receive the patient table 6 from a determined position in the respective treatment section or to discharge the patient table 6 from the support vehicle 14 to the determined position. The mechanical coupling and load/unload mechanism 34 provides means for adjusting the patient table 6 in a desired height, i.e. lower position for children, higher position for adults as indicated by an arrow 36.

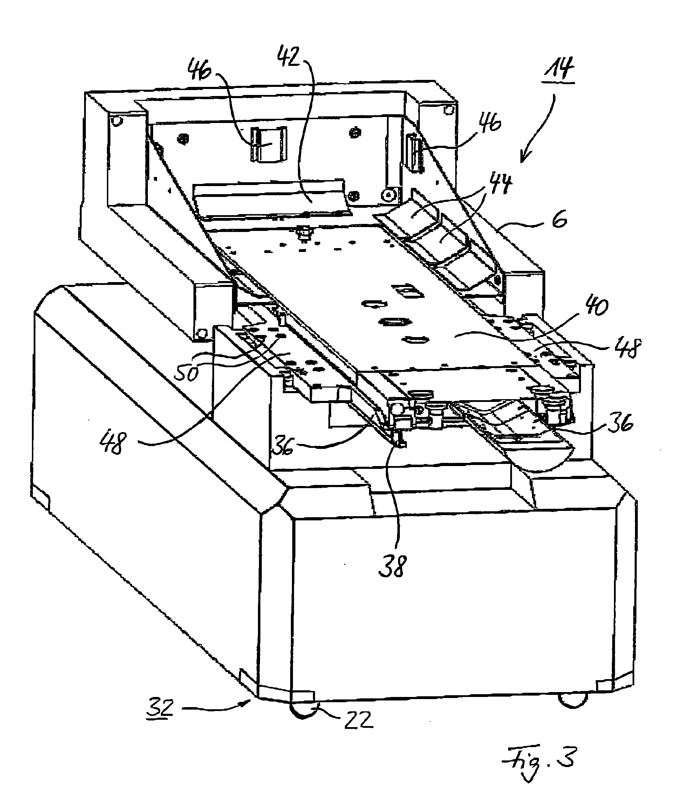

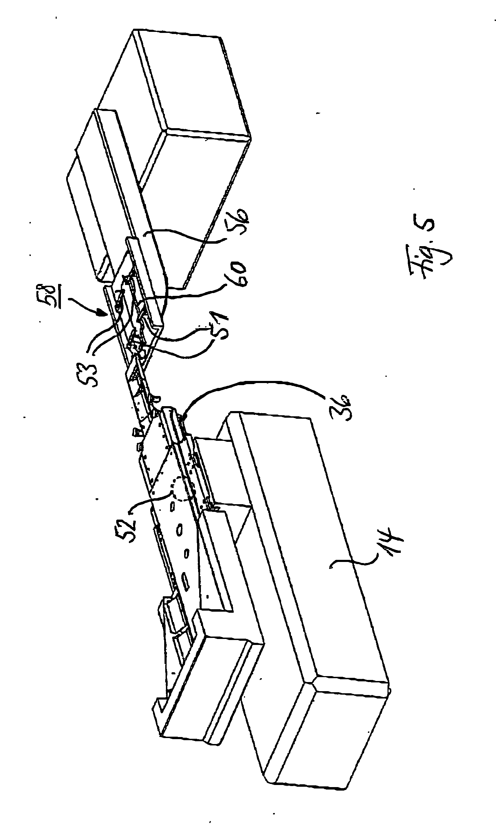

Figure 3 now illustrates in more detail the support vehicle 14 being loaded with the patient table 6 and the mechanical coupling and load/unload mechanism 34. The patient table 6 comprises a reclining area 40, reclining head pads 42 and reclining arm pads 44. Mounting elements 46 can be used to attach medical equipment, such as a drip infusion, to the patient table 6. The afore-mentioned mechanism 34 comprises support rails 38 which are L-shaped. At its front edge 38 the support rail 36 are flattened for allowing an easier positioning when the patient table 6 is unloaded into a predetermined position within the patient diagnosis and/or therapy section 10, 12. For locking the patient table 6 in the determined position locking sections 48 are provided with the patient table 6 which comprises boreholes 50 and cavities (not shown) disposed on the bottom side of the locking sections 48. The boreholes 50 and cavities cooperate with respective bolts 51 (Figure 5) penetrating the boreholes 50 and respective locking hooks 53 (Figure 5) penetrating the cavities when the patient table 6 is either unloaded into the predetermined position in the treatment section or re-loaded from the predetermined position within the treatment section back to the target position on the support vehicle 14.

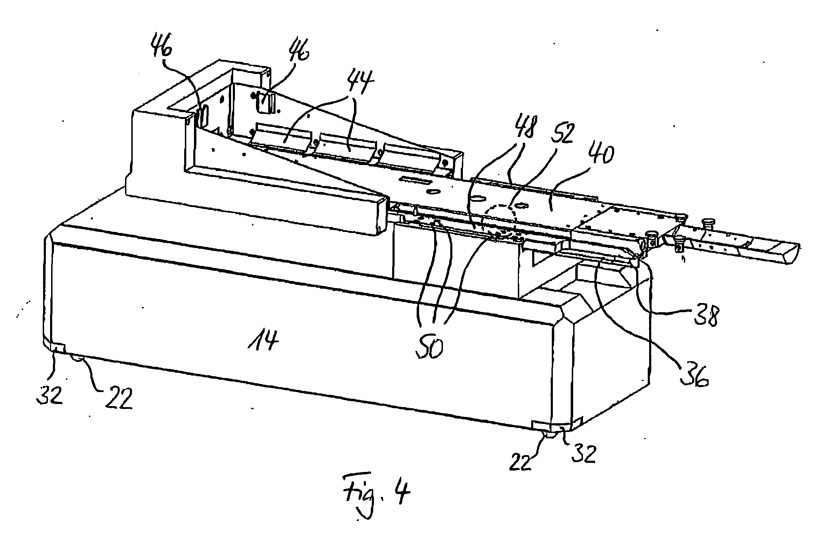

For loading and unloading the patient table 6 the support vehicle 14 comprises an electrically driven rubber role 52 which is shown in Figure 4 as a dotted circle. This rubber role 52 is in a friction contact with a contact surface on the bottom area of the patient table 6. Further with Figure 4, the front edges 38 of the rails 36 can be recognized again as being flattened to improve the introduction of the rails 36 in the respective ducts 60.

with reference to Figure 5 a situation is shown where the patient table 6 is unloaded to a pivot arm 56 of a proton therapy gantry. When the support vehicle is positioned (self-guided) properly relative to the pivot arm 56, the rubber role 52 drives the patient table 6 towards a receiving section 58 comprising the afore-mentioned bolts 51 and hooks 53. The receiving section 58 is further comprising the ducts 60 in which the rails 36 are inserted during the unloading. The front sections of the ducts 60 are shaped as dove-tails in order to support the introduction of the rails 36 (starting with its flattened edges 38) into the ducts 60. When the patient table 6 has reached its target position on the pivot arm 56, both the bolts 51 and the hooks 53 are automatically inserted into the respective boreholes 50 and cavities. The patient table 6 is now locked to the pivot arm and can be operated for the desired proton therapy. The therapy program controls then the pivot arm 56 with the patient table 6 accordingly.

After the therapy session, the patient table 6 is re-loaded to the support vehicle 14. Therefore, the support vehicle 14 is again positioned properly and the rubber roll 52 pulls the patient table 6 back into the target position on the support vehicle after the bolts 51 and hooks 53 have been released.

In summarizing the present invention, the patient transport system 2 allows to position a patient on a patient table 6, 6a, 6b and to transport the patient table 6, 6a, 6b supported on the support vehicle 14 first to a preparation station 4 (where the patient of course could also enter the patient table) and afterwards to the intended diagnosis and/or therapy section 10 and 12 and 20 resp. At these sections the patient table 6, 6a, 6b is discharged from the support vehicle 14 into a predetermined position within the diagnosis and/or therapy section. Additionally, after the diagnosis and/or therapy treatment, the patient table 6, 6a, 6b is again received on the support vehicle 14 and, subsequently, the support vehicle 14 is directed back to the preparation section 4 or to an after treatment section 20 where the patient can finally leave the patient table 6, 6a, 6b and return to his sick room either by feet or in his sick bed. By having the concept of the support vehicle 14 which receive and discharge the patient tables 6 the patients can be prepared in a slot-like chain according the treatment schedule chosen for the diagnosis and/or therapy section which allow a high throughput of patients.

## Claims

1. A patient transport system (2), comprising:
a) at least one patient table (6, 6a, 6b);
b) at least one self-propelled support vehicle (14) having means (34, 36, 38, 50, 51, 52, 53) for loading a patient - table (6, 6a, 6b) and/or means for unloading a patient table (6, 6a, 6b);
c) a patient diagnosis and/or therapy station (10, 12); and
d) a control means (28, 30) for controlling the operation of the at least one self-propelled support vehicle (14); wherein
e) a patient disposed on the patient table (6, 6a, 6b) is presentable at the patient diagnosis and/or therapy station (10, 12) by controllably moving the support vehicle (14); and
f) said means for unloading a patient table (6, 6a, 6b) are provided to unload the patient table (6, 6a, 6b) from the support vehicle (14) into a determined position within the patient diagnosis and/or therapy station (10, 12, 20) and/or said means for loading the patient table (6, 6a, 6b) are provided to load the patient table (6, 6a, 6b) from the determined position within patient diagnosis and/or therapy station (10, 12, 20) into a determined position on the support vehicle (14).

2. The system according to claim 1, wherein a patient preparation station (4) is provided and the patient disposed on the patient table (6, 6a, 6b) is presentable at the patient preparation station (4).

3. The system according to claim 1 or 2, wherein a patient loading station (4) is provided comprising means (34) for disposing the patient table (6, 6a, 6b) in different heights.

4. The system according to claim 1 or 2, wherein the means (34) for unloading and/or loading the patient table (6, 6a, 6b) comprises positioning aids (38, 60) in order to shift the patient table (6, 6a, 6b) into the determined position on the support vehicle (14) as well as in the preparation station (4) and/or in the patient diagnosis and/or therapy station (10, 12, 20).

5. The system according to any of the preceding claims, wherein monitoring and/or patient supply equipment (26) is supported by the patient table (6, 6a, 6b) and/or the support vehicle (14).

6. The system according to claim 4, wherein said monitoring and/or patient supply equipment is at least one unit selected from a group comprising of a ECG unit, a blood pressure unit, an anaesthetic supply unit, a lung ventilator unit and a heart pulse rate counter.

7. The system according to any of the preceding claims, wherein the patient table (6, 6a, 6b) comprises electrical supply (24) and connecting means in order to be supplied with electric power in the patient preparation station (4) and/or while the patient table (6, 6a, 6b) is transported on the support vehicle (14) and/or in the patient diagnosis and/or therapy station (10, 12, 20).

8. The system according to any of the preceding claims, wherein the support vehicle (14) comprises guidance means (32) in order to commute self-guided to a determined position.

9. The system according to claim 7, wherein the guidance means comprise path finding sensors (32) which interact with respective landmarks (16) positioned along the commuting paths.

10. The system according to claim 7, wherein the guidance means comprise acceleration and direction sensors and/or wireless navigation sensors in order to self-guiding the support vehicle along the desired commuting path.

11. The system according to any of the preceding claims, wherein the patient table (6, 6a, 6b) comprises control means for monitoring the position of the patient disposed on the patient table.

12. The system according to claim 10, wherein the control means comprise position sensors using ultrasonic position detection and/or using optical position detection and/or using RFID proximity detection.

13. The system according to claim 10 or 11, wherein the signals from the control means are checked against predetermined threshold values whereby diagnosis and/or therapy application is suspended in case one or more threshold values are exceeded.

14. A patient transport system (2), comprising:
a) at least one patient table (6, 6a, 6b);
b) at least one self-propelled support vehicle (14) having means (34, 36, 38, 50, 51, 52, 53) for loading a patient table (6, 6a, 6b) and/or means for unloading a patient table (6, 6a, 6b);
c) a patient diagnosis and/or therapy station (10, 12); and
d) a control means (28, 30) for controlling the operation of the at least one self-propelled support vehicle (14); wherein
e) a patient disposed on the patient table (6, 6a, 6b) is presentable at the patient diagnosis and/or therapy station (10, 12) by controllably moving the support vehicle (14);
